# EUROPEAN PATENT APPLICATION

(11) **EP 1 958 635 A1**
(43) Date of publication of application: **20.08.2008**
(21) Application number: 08003109.9
(22) Date of filing: 30.12.2004
(51) Int. Cl.: A61K 31/57, A61K 9/00, A61P 25/24

(54) **Treatment**

(30) Priority: 03.01.2004 GB 0400031
(62) Divisional of application: 04806214.5
(71) Applicant: Diurnal Ltd, 217 Portobello Sheffield S1 4DP (GB)
(72) Inventor: Ross, Richard, Glossop Road Sheffield, S10 2JF (GB)
(74) Representative: Gilholm Harrison Ltd

(57) **Abstract**

We describe a treatment regime for the treatment of conditions which result from the abnormal secretion of cortisol.

## Description

The invention relates to the treatment of conditions, for example depression, which would benefit the delivery of a delayed and sustained release formulation of hydrocortisone, or variant thereof, to entrain cortisol secretion in a circadian rhythm.

In mammals the principle oscillator of circadian rhythms or central clock is in the suprachiasmatic nucleus. This master oscillator is responsible for the sleep-wake cycle and hormonal rhythms (e.g. cortisol and melatonin). It is recognised that peripheral tissues such as immune cells and the liver also have clock genes and there own molecular oscillating ability. We herein disclose that the circadian rhythm of cortisol is the secondary messenger that sets peripheral tissue clock time according to the central clock in the suprachiasmatic nucleus.

Disturbance of the central clock may occur as a result of either an environmental change (an individual moves to a different time zone, does shift work, or change in season) or as a result of disease either directly effecting the central oscillator or altering the circadian rhythm or the secondary messenger cortisol. The loss of circadian rhythms results in diseases for example depression and inflammatory disorders. Thus, diseases such as inflammation and depression can cause a loss of circadian rhythms and loss of circadian rhythms can cause disease such as depression. For example it is well recognised that depression and rheumatoid arthritis are associated with loss of the normal cortisol circadian rhythm e.g. disease effecting rhythm. Similarly it is well recognised that Cushing's syndrome where a tumour results in loss of the circadian rhythm of cortisol causes depression e.g. loss of circadian rhythm causes disease.

An example of a peripheral tissue that has a circadian rhythm is the immune system. The immune response as characterised by cytokine levels has a circadian rhythm. For example: maximal expression of T helper 1 cytokines coincide with the nocturnal nadir of plasma cortisol, whereas T helper 2 cytokine responses are dominant during day-time. Many inflammatory diseases such as rheumatoid arthritis and asthma have a distinct circadian being worse first thing in the morning as consequence of the disturbed circadian of the immune response.

Daily rhythms in gene expression, physiology, and behaviour persist under constant conditions and must, therefore, be driven by self-sustained biological oscillators called circadian clocks [for reviews, see 1. J. C. Dunlap, Cell 96, 271 (1999). 2. S. A. Brown and U. Schibler, Curr. Opin. Genet. Dev. 9, 588 (1999).]. Circadian clocks can count time only approximately and must be adjusted every day by the photoperiod in order to be in harmony with the outside world. In mammals, light signals perceived by the retina are transmitted directly to the suprachiasmatic nucleus (SCN) via the retino-hypothalamic tract [M. H. Hastings, Curr. Biol. 7, 670 (1997).] The SCN, located in the ventral part of the hypothalamus, is thought to contain the master pacemaker, which synchronizes all overt rhythms in physiology and behavior [B. Rusak and I. Zucker, Physiol. Rev. 59, 449 (1979).].

Molecular oscillators exist in most peripheral cells of mammals [A. Balsalobre, F. Damiola, U. Schibler, Cell 93, 929 (1998). S. Yamazaki et al., Science 288, 682 (2000).] In mammals, it is thought that the phase of these peripheral time-keepers is reset by signals regulated by the SCN pacemaker [S. Yamazaki et al., Science 288, 682 (2000).] Because serum induces circadian gene expression in cultured rat-1 fibroblasts [A. Balsalobre, F. Damiola, U. Schibler, Cell 93, 929 (1998).] one or more blood-borne factors must stimulate signal transduction pathways that influence the molecular oscillators in peripheral cells. Glucocorticoid hormones are particularly attractive candidates, because (i) they are secreted in daily cycles and (ii) the glucocorticoid receptor (GR) is expressed in most peripheral cell types, but not in SCN neurons [P. Rosenfeld, J. A. M. Van Eekelen, S. Levine, E. R. De Kloet, Dev. Brain Res. 42, 119 (1988).] The second point would be consistent with glucocorticoids as entraining signals, given that circadian gene expression has a different phase angle in the SCN and in peripheral tissues [L. Lopez-Molina, F. Conquet, M. Dubois-Dauphin, U. Schibler, EMBO J. 16, 6762 (1997).].

Using dexamethasone, which does not affect the central circadian pacemaker in the SCN, it has been shown that peripheral oscillators can be phase delayed or phase advanced during the entire 24-hour day. This phase-shifting behaviour would be expected for slave oscillators that are synchronized by a master pacemaker because they should remain responsive to phase-resetting signals from the SCN at all times [Balsalobre et al., Science 289, 2344 (2000)]. These results suggest that glucocorticoids could provide the secondary messenger from SCN to most mammalian tissues.

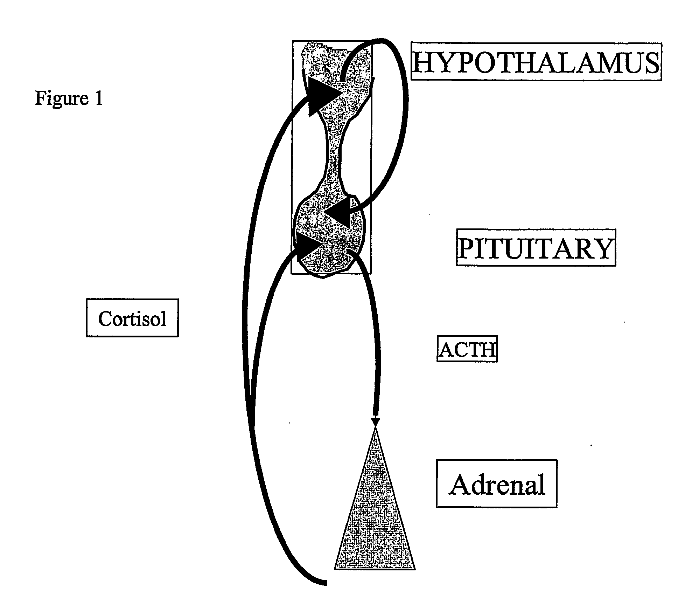

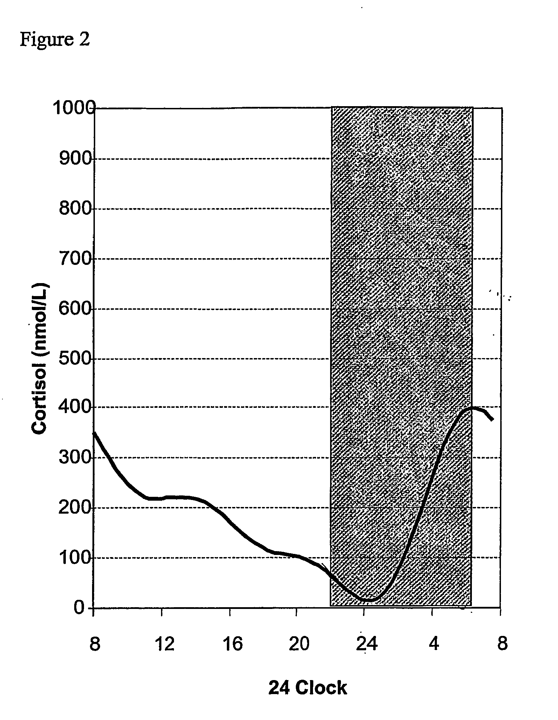

Biological clocks in tissues that are regulated by glucocortiocoids, for example cortisol, could include the brain, endocrine, immune system, lungs, cardiovascular system, genitor-urinary system, reproductive system. Cortisol, also called the "stress hormone", is secreted by the adrenal glands which are adjacent the kidneys. Cortisol secretion increases when the body is stressed, either physically or psychologically. Hydrocortisone is the most commonly used drug as it is equivalent to cortisol, is rapidly absorbed and is inexpensive. Cortisol is released from the adrenal gland under the regulation of ACTH derived from the pituitary gland (Fig 1). There is a circadian rhythm to cortisol release with high levels first thing in the morning and very low levels around midnight (Fig 2). ACTH and thus cortisol levels begin to rise around 3am and peak at 7am gradually falling over the day to a nadir at midnight (Krieger *et al.,* 1971; Ross *et al.,* 1991).

Conditions which result in depression or a general malaise are well known. These include, but are not limited to, clinical depression, reactive depression, post-natal depression, depression which results as a consequence of extensive surgery, chronic fatigue, myalgic encephalitis and conditions such as jetlag. Depressive conditions can also be induced by external effects which are self imposed, for example depression can result when a person is either dieting in an effort to lose weight and also when a person is attempting to treat an addiction, for example smoking or cocaine addiction. There are also less serious conditions which result in general malaise, for example shift workers who work unsociable hours can become tired and depressed due to disruption in their normal sleep pattern. It is also known that certain periods of the year induce depression, for example Christmas and Seasonal Affective Disorder (SAD).

The physiological effects of depression are varied. However, some general characteristics include, hyperactivity of the hypothalamo-pituitary-adrenal axis resulting in high cortisol levels, loss of the cortisol circadian rhythm and sleep disturbance with early morning waking. High glucocorticoid levels at night-time or at the time of going to sleep may also themselves disturb sleep. It is known that antagonistic drugs that lower cortisol levels alleviate the symptoms of depression.

For example corticotrophin releasing factor (CRF), (the hypothalamic hormone that induces ACTH release which in turn induces cortisol release) antagonists have been investigated as potential therapies for depression.

Furthermore, specific antagonists of cortisol activity are known in the art. WO9917779 describes the use of glucocorticoid receptor antagonists to ameliorate psychotic disorders. Cortisol is a glucocorticoid which binds an intracellular glucocorticoid receptor and elevated levels of cortisol, or hypercortisolemia, can be controlled by blocking the activity of the receptor to which cortisol binds. An example of a cortisol receptor antagonist is mifepristone and WO9917779 teaches the use of this antagonist to treat conditions that result from elevated cortisol levels. A further example is described in WO02076390 which teaches the use of glucocorticoid receptor antagonists to treat stress conditions, for example post traumatic stress disorder, in individuals.

In EP1201244 the lowering of elevated cortisol levels is achieved by administration of a composition which alleviates depression and mental stress. The composition comprises a complex of phosphatidyl-L-serine and phosphatidic acid which lowers cortisol and increases serotonin to normal levels.

US2003027802 provides a yet further example of a treatment which alleviates the symptoms of depression which arise as a consequence of weight gain induced by anti-psychotic medications (e.g clozapine, olanzapine, risperidone, quetiapine or sertindole). This is a second medical indication for cortisol receptor antagonists and in particular mifepristone.

What is apparent from the above is that medications which are currently used to treat depression and related disorders are primarily inhibitors of cortisol activity, either directly, or indirectly by blocking receptor activation of a glucocorticoid receptor.

Delivery vehicles which can be used in the working of the invention are known in the art. For example, US 4,261,969, which is incorporated by reference, discloses a polymer composition which is enzyme activated. The composition comprises a sensing means which can detect small amounts of a compound in a complex mixture, for example blood, which is an indicator of the body's need for the drug, and a delivery means which senses the change in the sensing means thereby releasing the drug at a required time in a dose dependent manner. The system is suitable for use in the delivery of a contraceptive drug. A further example is disclosed in EP01077065, which is incorporated by reference. The controlled release formulation comprises a drug core which is surrounded by a release control layer which breaks down after a predetermined delay. EP01077065 also discloses a drug release layer outside the release control layer which provides for an initial rapid release followed by the release of drugs from the drug core. This provides for the delivery of at least two drug doses in a delayed manner.

US 6, 207, 197, which is incorporated by reference, discloses a pharmaceutical composition which is adapted to be retained in the stomach to treat diseases, such as ulcers. These are referred to as gastro-retentive drugs. The invention describes microspheres comprising an inner core containing a therapeutic agent surrounded by a water insoluble polymer which is provided with an outer layer of bioadhesive cationic polymer. The adhesive polymer functions to retain the microsphere in the stomach thereby facilitating the concentrated release of the therapeutic agent in the stomach. EP01053752, which is incorporated by reference, discloses a further example of a preparation which shows controlled release. The preparation comprises two parts, a female and male part, the female part is made from a water insoluble polymer and the male part formed from a composition consisting of ethyl acrylate: methyl methacrylate: trimethylammonioethyl methacrylate co-polymer and a methylacrylic:ethyl acrylate co-polymer. The therapeutic agent is contained within the male and female parts. The formulation is pH sensitive only releasing a therapeutic agent at neutral pH thereby passing through the stomach intact and only releasing in the neutral environment of the small intestine.

WO010957, which is incorporated by reference, discloses an implant which is provided with a coating comprising a polymer matrix which is formed from ethylenically unsaturated monomers which includes a zwitteronic monomer, for example 2-methacryloyloxyethyl-2'-trimethylammoniumethlyphosphate salt. The composition absorbs a therapeutically active substance that is then dried by evaporation of the solvent included with the active substance. The implant is then ready for implantation into the patient and begins to slowly release the active substance. US 6, 217, 911, which is incorporated by reference, discloses a controlled release microcapsule for the controlled release of non-steroidal antiinflammatory drugs for 24 hours to 2 months. The microcapsule is biocompatible and biodegradable and manufactured from DL-lactide-co-glycolide. The composition is topically applied to soft tissues surrounding a surgical incision or wound site. Typically the microspheres are loaded with lidocaine to provide slow release pain relief.

A yet further example of a delayed release formulation is disclosed in WO02/30398, which is incorporated by reference in its entirety. The formulation comprises a core which includes a drug and a disruption agent and further comprises a regulatory membrane coating on the core formed from a mixture of a water soluble gel-forming polymer and a water -insoluble film-forming polymer. The disruption agent is for example an agent which expands on hydration (e.g. hydroxypropylcellulose, sodium starch glycolate, sodium carboxymethylcellulose, croscarmellose sodium or carbomer). The core inlcudes a spheronisation aid (e.g. microcrystalline cellulose). The water soluble gel forming polymer of the regulatory membrane coating is a high viscosity grade hydroxyalkyllcellulose (e.g. hydroxypropylmethylcellulose) or a methyl cellulose. The water insoluble film forming polymer of the regulatory membrane coating is alkyl cellulose (e.g. ethyl cellulose).

The generation of a delayed and sustained form of hydrocortisone provides a tool to administer circadian cortisol. By changing the time of application one can delay, advance, entrain the body's circadian rhythm and through this mechanism treat diseases where there is disorder in the biological clock resulting in depression, sleep disorder, fatigue, abnormal eating, addiction, anxiety, immune response, inflammation, arthritis, jet lag.

In our co-pending application, WO03/015793, we describe the treatment of adrenal dysfunction by the provision of a hydrocortisone composition which has the characteristics of delayed and sustained release and which corrects defects in the circadian release of cortisol. We describe, in the present application, a treatment regime which utilises the delayed and sustained release characteristics of the composition described in WO03/015793 in the treatment of depression and related conditions that would benefit from entrainment of cortisol secretion. The treatment, rather than reduce cortisol or cortisol activity, as is taught in the prior art, produces its effect by returning the patients circadian levels of cortisol to normal by entrainment thereby alleviating the symptoms of depression as a consequence of abnormally high cortisol concentrations. The invention therefore provides a treatment regime suitable for an animal which comprises the administration of at least hydrocortisone and means which allow for the delayed and sustained release of hydrocortisone for the treatment of conditions that would benefit from the circadian entrainment of cortisol secretion.

According to an aspect of the invention there is provided the use of a combined composition comprising hydrocortisone and a delivery vehicle wherein said delivery vehicle provides for delayed and sustained release of hydrocortisone, for the manufacture of a medicament for use in the treatment of diseases or conditions that result from disruption of the normal circadian secretion of cortisol.

The central clock oscillator drives a very precise circadian rhythm of cortisol with a nadir around 2am and peak around 7am. The rise in cortisol starts around 2 to 3am. By administering a delayed and sustained hydrocortisone between the hours of 1800 to 2400h we can reset and re-entrain the circadian rhythm of cortisol. For example, if we phase shift the administration forward and create an earlier build up in cortisol this will prevent the switch on of the normal clock genes that trigger the rise in cortisol if we then relax the phase by taking the tablet later this will allow the normal circadian rhythm to be established. For example, in a depressed patient there is loss in the circadian rhythm of cortisol. The patient will be given a tablet of delayed and sustained cortisol at 2100h that results in rise in cortisol at 0100h before the normal rise in cortisol. This will switch off the endogenous rise in cortisol through negative feedback. Thus a circadian rhythm is established and then over time the administration of delayed and sustained hydrocortisone can be reduced and give later to allow the reappearance of a normal entrained circadian rhythm of cortisol. The same process can be used to treat other conditions, for example inflammatory disorders. A further feature of the treatment regimen maybe the requirement to give supraphysiological doses of hydrocortisone in a phase advanced cycle to allow entrainment of the circadian cortisol rhythm.

Diseases or conditions that would benefit from entrainment of cortisol secretion include but are not limited to, depression, sleep disorder, fatigue, abnormal eating, addiction, anxiety, immune response, inflammation, arthritis, asthma, jet lag.

In a preferred embodiment of the invention said use is the treatment of depression or a condition which results in depression, or similar condition.

In a preferred embodiment of the invention the depression is clinical depression.

In a further preferred embodiment of the invention the depression is reactive depression.

In a still further preferred embodiment of the invention the depression is post-natal depression.

In a further preferred embodiment of the invention said condition or malaise is selected from the following group: chronic fatigue syndrome; myalgic encephalitis; jet lag; shift work, depression resulting from a person being overweight; depression resulting from dieting; depression resulting from the treatment of cigarette addiction; depression resulting from the treatment of alcohol addiction; depression resulting from the treatment of drug addiction (e.g. cocaine, heroin); the treatment of seasonal affective disorder or a like condition.

In a further preferred embodiment of the invention said condition results from the administration of an antipsycotic drug.

In a preferred method of the invention said antipsycotic drug is selected from the group consisting of: clozapine, olanzapine, risperidone, quetiapine or sertindole.

When administered, the compositions of the present invention are administered in pharmaceutically acceptable preparations. Such preparations may routinely contain pharmaceutically acceptable concentrations of salt, buffering agents, preservatives, compatible carriers, and optionally other therapeutic agents useful in the treatment of depression or the like.

The composition of the invention can be administered by any conventional route, including injection. The administration may, for example, be oral, intravenous, intraperitoneal, intramuscular, intracavity, subcutaneous, or transdermal.

The compositions of the invention are administered in effective amounts. An "effective amount" is that amount of a composition that alone, or together with further doses, produces the desired response. This may involve only slowing the progression of the disease temporarily, although more preferably, it involves halting the progression of the disease permanently. This can be monitored by routine methods or can be monitored according to diagnostic methods.

The compositions used in the foregoing methods preferably are sterile and contain an effective amount of cortisol/hydrocortisone, glucocorticoid or derivatives thereof for producing the desired response in a unit of weight or volume suitable for administration to a patient. The response can, for example, be monitored by measuring the physiological effects of the composition, such as a decrease of disease symptoms and/or measurement of ACTH and cortisol levels where appropriate. Assays will be known to one of ordinary skill in the art and can be employed for measuring the level of the response.

The doses of the composition administered to a subject can be chosen in accordance with different parameters, in particular in accordance with the mode of administration used, the time of dosing and the state of the subject (i.e. age, sex, weight, body mass index (BMI)). Other factors include the desired period of treatment. In the event that a response in a subject is insufficient at the initial doses applied, the time of dosing maybe varied and higher doses (or effectively higher doses by a different, more localized delivery route) may be employed to the extent that patient tolerance permits.

In general, doses of the composition are formulated and administered in doses between 1 mg and 30 mg, and preferably between 10mg and 25mg, according to any standard procedure in the art. More preferably still said sustained release composition is administered between 1 mg and 30mg at night and between 1 and 15 mg in the morning. In certain patients an increased dosage may be desirable, for example dosages of between about 100mg-200mg of hydrocortisone. The timing of administration is in accordance with consultation with the patient's doctor. Typically dosages may be administered between 18:00 and 24:00.

An animal as used herein is a mammal, preferably a human, and including a non-human primate, cow, horse, pig, sheep, goat, dog, cat or rodent.

When administered, the compositions of the invention are applied in pharmaceutically-acceptable amounts and in pharmaceutically-acceptable compositions. Such preparations may routinely contain salts, buffering agents, preservatives, compatible carriers, and optionally other therapeutic agents. When used in medicine, the salts should be pharmaceutically acceptable, but non-pharmaceutically acceptable salts may conveniently be used to prepare pharmaceutically-acceptable salts thereof and are not excluded from the scope of the invention. Such pharmacologically and pharmaceutically-acceptable salts include, but are not limited to, those prepared from the following acids: hydrochloric, hydrobromic, sulfuric, nitric, phosphoric, maleic, acetic, salicylic, citric, formic, malonic, succinic, and the like. Also, pharmaceutically-acceptable salts can be prepared as alkaline metal or alkaline earth salts, such as sodium, potassium or calcium salts.

The compositions may be combined, if desired, with a pharmaceutically-acceptable carrier. The term "pharmaceutically-acceptable carrier" as used herein means one or more compatible solid or liquid fillers, diluents or encapsulating substances which are suitable for administration into a human. The term "carrier" denotes an organic or inorganic ingredient, natural or synthetic, with which the active ingredient is combined to facilitate the application. The components of the pharmaceutical compositions also are capable of being co-mingled with the molecules of the present invention, and with each other, in a manner such that there is no interaction which would substantially impair the desired pharmaceutical efficacy.

The compositions may contain suitable buffering agents, including: acetic acid in a salt; citric acid in a salt; boric acid in a salt; and phosphoric acid in a salt. The compositions also may contain, optionally, suitable preservatives, such as: benzalkonium chloride; chlorobutanol; parabens and thimerosal.

The compositions may conveniently be presented in unit dosage form and may be prepared by any of the methods well-known in the art of pharmacy. All methods include the step of bringing the active agent into association with a carrier which constitutes one or more accessory ingredients. In general, the compositions are prepared by uniformly and intimately bringing the active compound into association with a liquid carrier, a finely divided solid carrier, or both, and then, if necessary, shaping the product.

Compositions suitable for oral administration may be presented as discrete units, such as capsules, tablets, lozenges, each containing a predetermined amount of the active compound. Other compositions include suspensions in aqueous liquids or non-aqueous liquids such as a syrup, elixir or an emulsion.

Compositions suitable for parenteral administration conveniently comprise a sterile aqueous or non-aqueous preparation of cortisol/hydrocortisone or functional derivative thereof, which is preferably isotonic with the blood of the recipient. This preparation may be formulated according to known methods using suitable dispersing or wetting agents and suspending agents. The sterile injectable preparation also may be a sterile injectable solution or suspension in a non-toxic parenterally-acceptable diluent or solvent, for example, as a solution in 1,3-butane diol. Among the acceptable solvents that may be employed are water, Ringer's solution, and isotonic sodium chloride solution. In addition, sterile, fixed oils are conventionally employed as a solvent or suspending medium. For this purpose any bland fixed oil may be employed including synthetic mono-or di-glycerides. In addition, fatty acids such as oleic acid may be used in the preparation of injectables. Carrier formulation suitable for oral, subcutaneous, intravenous, intramuscular, etc. administrations can be found in Remington's Pharmaceutical Sciences, Mack Publishing Co., Easton, PA.

According to a further aspect of the invention there is provided a method for the treatment of an animal which animal is suffering from, or has a predisposition to depression, or a similar condition, comprising the steps of:
i) providing a combined preparation of hydrocortisone, or a functional variant thereof, and a delivery vehicle wherein said vehicle provides for the sustained release of hydrocortisone or variant thereof;
ii) administering the combined preparation in (i) to an animal requiring treatment such that hydrocortisone, or variant thereof, is released in a sustained manner;
iii) providing a combined preparation of hydrocortisone, or variant thereof, and a delivery vehicle wherein said vehicle provides for the delayed but sustained release of hydrocortisone, or variant thereof; and
iv) administering the combined preparation in (iii) to an animal requiring treatment such that hydrocortisone, or variant thereof, is released in a delayed but sustained manner.

In a further preferred method of the invention an animal is administered hydrocortisone which is released in a sustained manner which is followed by hydrocortsone which is released in a delayed but sustained manner.

In a preferred method of the invention said animal is human.

In a further preferred method of the invention said variant is cortisol or cortisone.

In a further preferred method of the invention said condition is selected from the group consisting of: chronic fatigue syndrome; myalgic encephalitis; jet lag; shift work; depression resulting from a person being overweight; depression resulting from a person dieting; depression resulting from the treatment of addiction, for example smoking or drugs (e.g. cocaine, heroine addiction); alcohol addiction; the treatment of seasonal affective disorder or a like condition.

In an alternative preferred method of the invention said condition results from the administration of an antipsycotic drug. Preferably said drug is selected from the group consisting of: clozapine, olanzapine, risperidone, quetiapine or sertindole.

A patient would take a sustained release preparation in the morning and a night-time preparation which would be a delayed and sustained release formulation. Based on pharmacokinetic modelling this twice daily administration would reproduce the normal circadian rhythm of, for example, cortisol production.

In a further preferred method of the invention said sustained release preparation is administered in the morning, preferably between 08:00 and 12:00.

In a yet further preferred method of the invention said delayed and sustained release formulation is administered in the evening, preferably between 18:00 and 24:00. More preferably between 20:00 and 24:00.

Moreover the administration of glucocorticoids in a delayed and sustained fashion will minimise the side-effects of glucocorticoids which are predominantly catabolic. Previous methods for preventing side-effects have focused on reducing systemic absorportion or reducing dose. For example, the use of delayed and sustained hydrocortisone would reduce side-effects because the administration replicates the circadian rhythm of cortisol thereby not exposing the body to excess cortisol at times when the body expects to have low levels and additionally by re-entraining the normal circadian rhythm disturbance of which results in unwanted side-effects such as immuno-suppression. Excess glucocorticoids result in increased abdominal fat, wasting of muscle, thin bones and skin, insulin resistance, and low levels of other anabolic hormones including IGF-I and sex steroids such as oestradiol and testosterone. Circadian glucocorticoid therapy will avoid a peak in glucocorticoid levels in the early phase of sleep when the body is fasted and catabolic. The normal build up of cortisol levels occurs prior to waking and by mimicking this circadian rhythm this therapy will prevent the catabolic complications of glucocorticoid therapy. Delayed and sustained glucocorticoid therapy will specifically reduce the deleterious effects of glucocorticoids on bone, skin, muscle, and fat mass.

An embodiment of the invention will now be described by example only and with reference to the following Figures:
Figure 1 represents a schematic representation of cortisol regulation in an animal;
Figure 2 represents the normal circadian rhythm of cortisol in an animal;

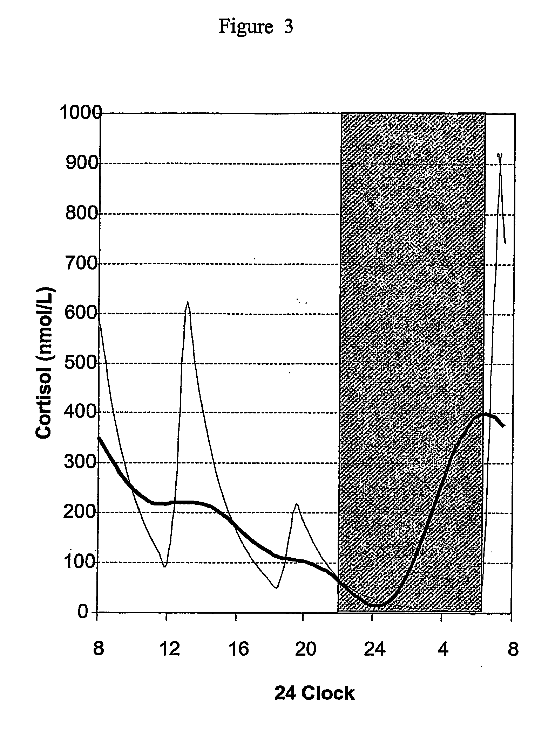

Figure 3 represents the cortisol profile in a patient taking hydrocortisone three times during a 24 hour period; and

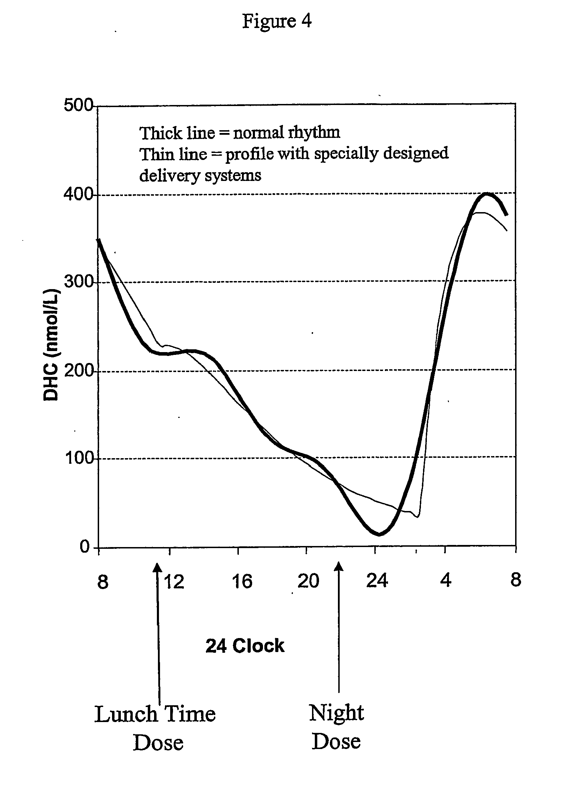

Figure 4 represents a cortisol profile in a patient taking a sustained and a sustained and delayed release composition of hydrocortisone;
Figure 5 illustrates ACTH and cortisol release in a normal individual;
Figure 6 illustrates cortisol secretion profile in a normal and depressed individual; and
Figure 7 illustrates the effect of delayed and sustained hydrocortisone on endogenous cortisol levels.

### Materials and Methods

### Problem in depression

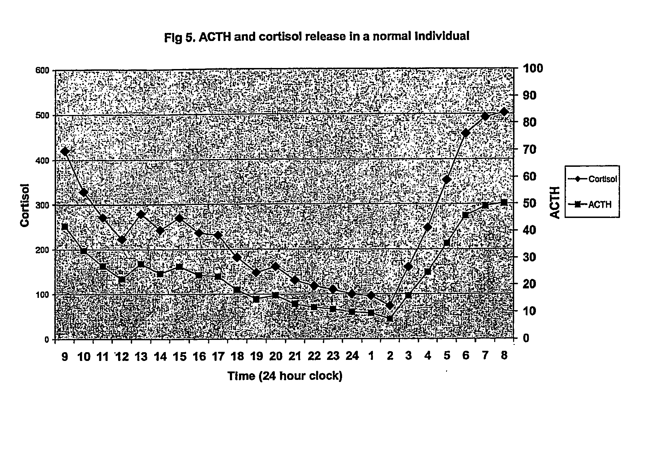

Patients with depression have increased cortisol release driven by ACTH and loss of the normal circadian rhythm of cortisol secretion. Cortisol release is entirely dependant on ACTH release from the pituitary. Thus, in the normal individual the release of cortisol parallels that of ACTH (Figure 5).

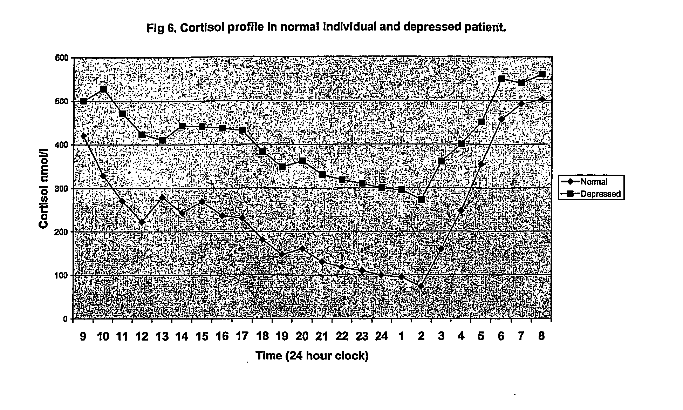

In depressed patients the overall cortisol release is greater and there is a failure of cortisol levels to fall to low levels at night. The profile of a depressed patient compared to a normal individual is illustrated in Figure 6. It is seen that the cortisol area under curve for the depressed patient is increased and the nadir cortisol level is high at midnight.

### Treatment of depression

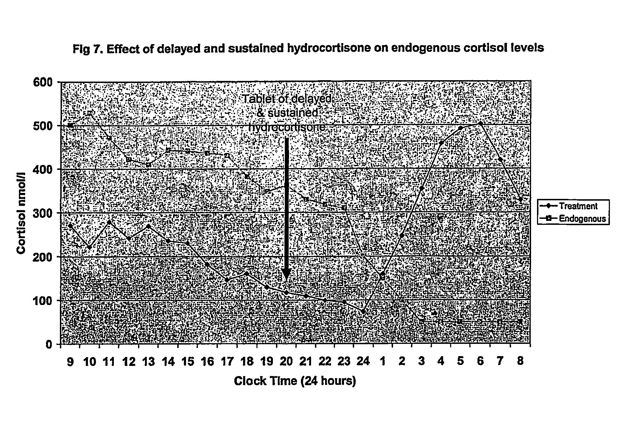

The solution to the problem is to entrain the normal circadian cortisol release. This is achieved by a gradual phase shift in the circadian rhythm of cortisol levels. Delayed and sustained hydrocortisone is given at a time to pre-empt the normal rise in cortisol levels. Thus, it is possible to switch off endogenous ACTH and cortisol levels. For example in a depressed patient the tablet may be given around, for example, 8pm such that the rise in cortisol levels will occur around midnight and prevent the expected rise in ACTH and cortisol levels at between and 2 and 3am. This is illustrated in Figure 7.

### Monitoring and entraining the normal circadian rhythm of cortisol in depression

Effective treatment will suppress endogenous ACTH and cortisol levels. The effect of treatment will be monitored by measuring the morning ACTH level. When this is suppressed the time of treatment can then be altered to bring the circadian cortisol profile back in phase. To achieve this, the tablet of delayed and sustained hydrocortisone may be given later in the evening to allow the rise in cortisol to occur between 2-3am. Again the success of therapy can be monitored by the measurement of ACTH. So the initial phase of treatment is to suppress the endogenous release of cortisol and the second phase is to entrain the normal circadian release of cortisol allowing low levels of cortisol at the time of going to sleep with the rise in cortisol levels occurring between 2-3 am and peaking on waking.

### Shift work and jetlag

Individuals undertaking shift work or moving through time zones do not have a circadian rhythm of cortsiol to match their sleep / wake pattern. Thus, they will often attempt to go to sleep with high levels of cortisol and wake with low levels of cortisol and fatigue. The solution is to take delayed and sustained hydrocortisone prior to sleeping. After 24 hours the delayed and sustained hydrocortisone will reinstitute the normal pattern of cortisol release until the sleep / wake cycle is reversed and which time the treatment may be stopped or the individual has established / entrained a new circadian rhythm of cortisol which usually takes 6 weeks.

## Claims

1. The use of a combined composition comprising a glucocorticoid, and a delivery vehicle wherein said delivery vehicle provides for delayed and sustained release said glucocorticoid for the manufacture of a medicament for use in the treatment of side effects due to the administration of said glucocorticoid.

2. Use according to Claim 1 wherein said glucocorticoid is selected from the group consisting of: hydrocortisone; cortisol, cortisone acetate, prednisolone, prednisone, or dexamethasone.

3. Use according to Claim 1 or 2 wherein said side effect is increasing weight gain.

4. Use according to Claim 1 or 2 wherein said side effect is reduction in bone density.

5. Use according to Claim 1 or 2 wherein said side effect is muscle or skin wastage.

6. Use according to Claim 1 or 2 wherein said side effect is insulin resistance.

7. Use according to any of claims 1-6 wherein said delayed and sustained release formulation is administered between 18:00 and 24:00.

8. Use according to claim 7 wherein said delayed and sustained release formulation is administered between 20:00 and 24:00.

9. Use according to any of claims 1-8 wherein said composition is suitable for oral administration.

10. Use according to claim 9 wherein said medicament is a capsule or tablet.

11. The use of a combined composition comprising hydrocortisone and a delivery vehicle wherein said delivery vehicle provides for delayed and sustained release of hydrocortisone, for the manufacture of a medicament to restore and entrain the endogenous circadian rhythm of cortisol in conditions that do not result from cortisol deficiency but are a consequence of a disruption in the normal circadian secretion of cortisol.
